# EUROPEAN PATENT APPLICATION

(11) **EP 0 927 558 A1**
(43) Date of publication of application: **07.07.1999**
(21) Application number: 97310694.1
(22) Date of filing: 31.12.1997
(51) Int. Cl.: A61K 47/10, A61K 47/26, A61K 9/16, A61K 51/12

(54) **Embolizing compositions comprising surfactants**

(71) Applicant: NYCOMED IMAGING AS, 0401 Oslo 4 (NO)
(72) Inventor: Bacon, Edward R., Audubon, PA19403 (US); Illig, Kathleen J., Phoenixville, PA19460 (US); Na, George C., Fort Washington, PA 19034 (US); Bessiére, Fabienne, 94100 Saint Maur (FR); McIntire, Gregory L., West Chester, PA 19382 (US)
(74) Representative: Cockbain, Julian, Dr.

(57) **Abstract**

This invention provides the use of a physiologically tolerable surfactant for the manufacture of an embolus generating pharmaceutical composition, comprising a suspension of a particulate embolic agent in a physiologically tolerable liquid suspension medium, for use in a method of therapy or diagnosis.

## Description

### FIELD OF THE INVENTION

The invention relates to the use of surfactants in the preparation of compositions for use in embolic therapy.

### BACKGROUND OF THE INVENTION

While emboli, stoppages of blood flow, are normally considered to be undesirable and sometimes are life-threatening, embolus generating agents have been used in certain fields of medical treatment, generally to block off blood supply to tumors or to tissue which is to be operated upon surgically. In the former case, embolization, optionally combined with chemotherapy (chemoembolization), achieves a beneficial cytotoxic effect. In the latter case, blood loss is reduced and surgery is facilitated. Current indications include occlusion of hypervascularized lesions, tumors, and arterio-venous malformations or shunts in neurological (head, neck, spine), visceral (kidney, stomach, lung) and limb regions. The embolus generating agent is usually administered via a catheter into an artery upstream of the site at which embolus formation is to occur. When injected into a vascular region, the embolus generating agents first mechanically occlude the vessel(s), and then induce blood stasis and thrombus organization.

Because embolus formation is generally undesirable, in embolus therapy it is particularly desirable that the embolus formed should be detectable by a diagnostic imaging modality (such as X-ray, MR imaging or ultrasound). However of the embolus generating agents currently in medical practice, only Lipiodol (Ethiodol) is amenable to imaging.

Lipiodol is an iodinated poppyseed oil which is administered as a water-in-oil emulsion and is observed by radiographic imaging to show where the embolus has localized. This approach however has the drawback that the oil droplets are susceptible to breaking up to form smaller droplets which may pass downstream of the target embolus site and cause emboli to form in tissues remote from the target organ, eg. in the lungs. As a result significant adverse events can result from this misdirected migration of the oily agent. The embolus may lodge too proximally to the intended site, allowing collaterisation of the target bed and may also translocate after an uncertain time. Thus with Lipiodol the behaviour of the embolic material in use cannot be accurately predicted.

Other conventionally used embolus generating agents, such as Ivalon and Gelfoam, are non-liquid particles which are not themselves detectable by imaging modalities and require the administration of a conventional water-soluble contrast agent (e.g., an X-ray agent such as Omnipaque) to enable the location of the embolus to be determined. This may be done by tracking the blood vessel of interest to detect the point at which contrast enhancement ceases.

With such non-liquid particulate embolus generating agents, hereinafter referred to simply as particulate embolic agents, it has been found that problems occur where even and measured administration through a catheter is required. Such problems are exacerbated as smaller and smaller selective (i.e., superselective) catheters are used. Thus while the current suspensions of particulate embolic agents in contrast media are stable, they present problems of agglomeration in the catheter such that the careful measurement of the amount of particles delivered and the controlled delivery of the particles are often problematic. To decrease the risk of clogging the catheter, physicians tend to use particulate embolic agents with a small average diameter. Such particles carry the risk of unwanted migration to and embolization of the normal vascular bed.

We have now found that these problems may be reduced or eliminated by the inclusion in the suspension to be administered of a physiologically tolerable surfactant.

### SUMMARY OF THE INVENTION

Thus viewed from one aspect the invention provides the use of a physiologically tolerable surfactant for the manufacture of an embolus generating pharmaceutical composition, comprising a suspension of a particulate embolic agent in a physiologically tolerable liquid suspension medium, for use in a method of therapy or diagnosis.

Viewed from a further aspect the invention provides a pharmaceutical composition comprising a suspension of a particulate embolic agent in a physiologically tolerable liquid suspension medium further containing a physiologically tolerable surfactant.

This embolus generating pharmaceutical composition according to the invention may particularly conveniently be produced by dispersion in a suspension medium, e.g., an X-ray or MR contrast medium, of a pre-mix, a composition comprising the surfactant and the particulate embolic agent. Such a premix forms a further aspect of the invention. Viewed from this aspect the invention provides a sterile pharmaceutical concentrate comprising a particulate embolic agent and a surfactant, preferably packaged in a sealed container.

Viewed from a still further aspect the invention also provides a method of treatment of the human or vascularized animal (e.g., mammal, reptile or bird) to create an embolus therein, said method comprising administering to said body, preferably through a catheter into the vasculature of the body, an effective amount of an embolus generating pharmaceutical composition according to the invention.

The embolus generating pharmaceutical composition of the invention may be administered more smoothly and precisely and will thus facilitate more accurate embolus placement. Such compositions will be particularly useful in oncology therapy and in minimizing bleeding during surgical procedures. The greater control over the amount of particulate embolic agent delivered to cause emboli arises as a result of the significant improvement in the flow of the particles through the catheter and the reduced tendency towards agglomeration in the catheter. Embolization of the target vasculature is not affected by the presence of surfactant.

### BRIEF DESCRIPTION OF THE DRAWINGS

Figure 1 is a graph showing the back pressure on the plunger of a syringe used to deliver a conventional embolus generating suspension at a rate of 0.3 ml/sec; and
Figure 2 is a graph showing the back pressure on the plunger of a syringe used to deliver an embolus generating pharmaceutical composition according to the present invention at a rate of 0.3 ml/sec.

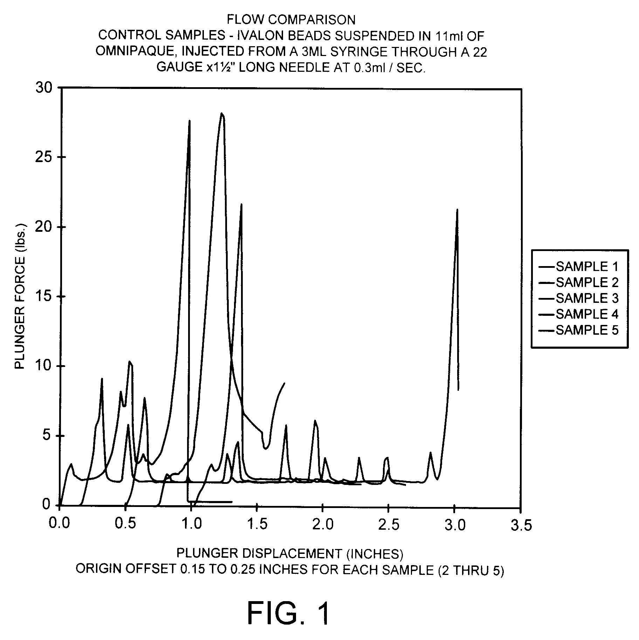

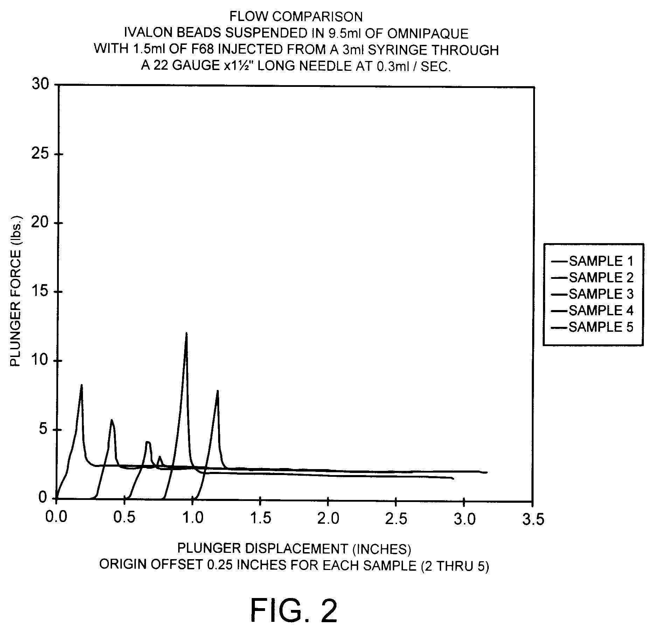

In Figures 1 and 2, the graphs are offset along the horizontal axis (Plunger displacement in inches) by 0.15 or 0.25 inches for successive samples so that the initial peaks do not overlap.

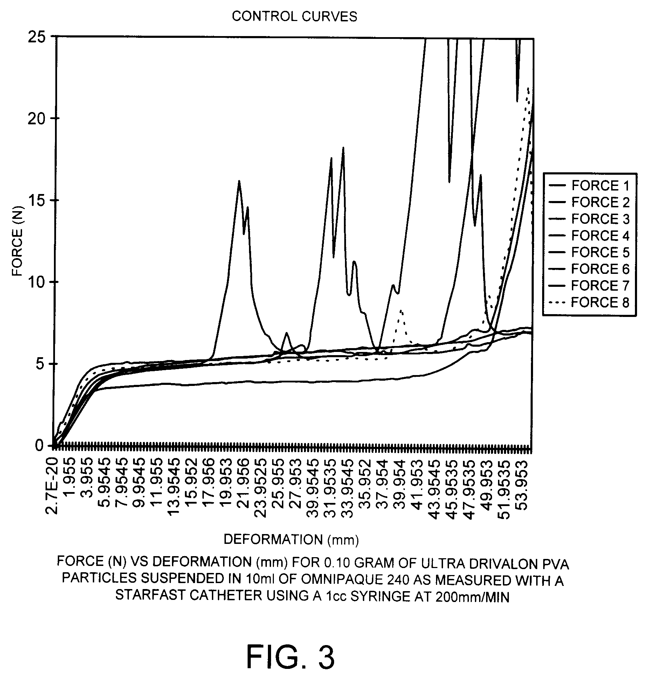

Figure 3 is a force displacement curve showing the fundamental forces present when a 1 cc syringe is used at 200 mm/min to deliver 0.1 g of Ultra Ivalon PVA particles suspended in 10 ml of Omnipaque 240 as measured with a StarFast catheter; and

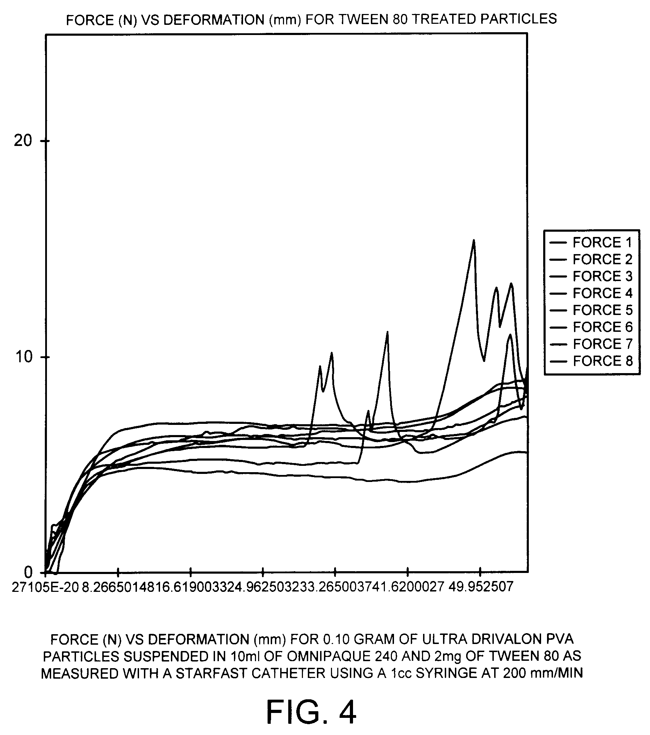

Figure 4 is a force displacement curve showing the fundamental forces present when a 1 cc syringe is used at 200 mm/min to deliver 0.1 g of Ultra Ivalon PVA particles suspended in 10 ml of Omnipaque 240 and 2 mg of Tween 80 as measured with a StarFast catheter.

### DETAILED DESCRIPTION OF THE INVENTION

Current embolus therapy involves placement of a catheter into the major blood vessel servicing the region of the cancerous lesion. At the appropriate point, the embolus causing pharmaceutical composition is administered through the catheter into the blood vessel to create the embolus at that point or at the first encounter of the particulate embolic agent with a region of that vessel which is sufficiently narrow as to result in the aggregation of the particulate embolic agent and the initiation of embolization. The pharmaceutical compositions of the invention may be similarly administered via catheter. The advantage of the compositions of the invention is that they are much easier to administer through the catheter with a greatly reduced tendency of the particulate embolic agent to agglomerate within the catheter itself. In addition, these compositions can include a conventional soluble contrast agent such as iohexol, iopentol, iodixanol, iopamidol, ioversol, etc. so that fluoroscopy can be used to follow the placement of the embolus in real time.

As shown in Figure 1, conventional PVA particles are often difficult to push through a restricted opening as judged by the tremendous build-up of pressure as the plunger is driven forward. These particles were diluted with Omnipaque as per the package insert and do not settle or float during the course of the experiment.

Figure 2 shows the same experiment with the addition of approximately 10 mg/ml of surfactant to ease the administration through the catheter. It is immediately clear that the pressure transients are singular (i.e., only one per push of the syringe plunger) and are much smaller than observed with the conventional materials. Again, the PVA particles were diluted with Omnipaque as per the package insert.

In the example of Figure 2, the surfactant used was Pluronic F-68 (i.e., Poloxamer 188). This material has been used in large volume parenterals and is generally regarded as safe (GRAS) for medical use. However other physiologically acceptable surfactants can be used, for example known organic and inorganic surfactants, for example polymers, low molecular weight oligomers, natural products and other surfactants. Preferred surfactants include nonionic and anionic surfactants. Representative examples include gelatin, casein, lecithin (phosphatides), gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., the commercially available Tweens), polyethylene glycols, polyoxyethylene stearates, colloidal silicon dioxide, phosphates, sodium dodecylsulfate, celluloses and cellulose esters (e.g., carboxymethylcellulose calcium, carboxymethylcellulose sodium, methylcellulose, hyroxypropylmethylcellulose phthalate, and noncrystalline cellulose), magnesium aluminum silicate, triethanolamine, polyvinyl alcohol (soluble), and polyvinylpyrrolidone (PVP). Most of these are described in detail in the Handbook of Pharmaceutical Excipients, published jointly by the American Pharmaceutical Association and the Pharmaceutical Society of Great Britain, the Pharmaceutical Press, 1986, the disclosure of which is hereby incorporated by reference in its entirety. The surfactants are commercially available and/or can be prepared by techniques known in the art.

Particularly preferred surfactants include poloxamers such as Pluronic F68, F98, F108, F127 and F88 (which are block copolymers of ethylene oxide and propylene oxide) and poloxamines such as Tetronic 908 and 1508 (which are tetrafunctional block copolymers derived from sequential addition of propylene oxide and ethylene oxide to ethylene diamine and are available from BASF, Parsippiny, NJ). In addition, block copolymers of butylene oxide and ethylene oxide are also acceptable in this application. Further, end group modified block copolymers of the Pluronics and Tetronic families and other like polymers may also be used herein. Other preferred surfactants include PVP, tyloxapol (Superinone), lecithin, and dialkyl esters of sodium sulfosuccinic acid such as Aerosol OT (which is the dioctyl ester of sodium sulfosuccinic acid and is available from American Cyanamid). Other classes of surfactants which are preferred include the Tweens and Spans. Tween 20, 40, 60 and 80 are polyoxyethylene sorbitan fatty acid esters while Span 20, 40, 60 and 80 are the corresponding sorbitan fatty acid esters. Two or more surfactants can be used in combination for this invention.

The surfactants may be a separate component of the compositions of the invention or alternatively they may be covalently bound to the surface of the particulate embolic agent.

The quantity of surfactant will depend upon the nature of the surfactant used and the quantity of particulate embolic agent used. Typically however the pharmaceutical composition will contain 0.0001 to 50 mg/ml, preferably 0.0002 to 1 mg/ml, especially 0.5 mg/ml surfactant and the premix will contain particles and surfactant in a weight ratio of 1:100 to 100:1, preferably 1:50 to 50:1.

The particulate embolic agent used in accordance with the invention may be any physiologically tolerable material capable of generating emboli. Although preferably water-insoluble, it may be poorly water-soluble such that the emboli remain in place for a period sufficient for the purposes of the treatment, i.e., generally a period of several hours at least. The particles may be organic or inorganic although organic materials and in particular polymers are preferred. Especially preferably they are polyvinyl alcohol, e.g., cross-linked polyvinyl alcohol particles, conveniently of the form available commercially as Ivalon or Ultra Ivalon, polyacrylonitrile or acrylic polymer. The particle size will depend on the desired placement of the emboli but will generally be in the range 0.005 µm to 1 mm, preferably 0.1 µm to 1 mm, especially 50 to 100 µm. The particles may be used at conventional concentrations, e.g., 2 to 60 g/l, preferably 8 to 40 g/l, most preferably 25 g/l.

The particles are preferably substantially uniformly sized, particularly preferably substantially monodisperse, e.g., with a coefficient of variation (mean particle size x 100/standard deviation) of less than 10%. However, more broadly dispersed particles are effective and improved with surfactant added.

The suspension medium is preferably aqueous and particularly preferably is a solution of a contrast agent present in a diagnostically detectable concentration. Accordingly, contrast agent solutions as supplied ready for injection may be used. Especially preferably the contrast agent is an x-ray contrast agent, e.g., an iodinated organic compound, more especially a triiodophenyl compound, e.g., iohexol, ioversol, iopamidol, iopentol or iodixanol. The x-ray contrast agent may typically be present at a concentration of 100 to 450, especially 200 to 350, mg I/ml.

The particulate embolic agent may preferably be treated with the surfactant and packaged, for dilution before use with a conventional contrast medium. If desired, the particulate embolic agent may serve as a carrier for a cytotoxic drug (e.g., carboplatin) which it will release at the site of the embolus, and advantageously the particles will be conjugated to a targeting vector, a material which facilitates the accumulation of the particles at sites within the body where embolus formation is desired. In this regard it is preferred to conjugate the particles to drugs or peptidic vectors which accumulate at areas of angiogenesis, e.g., peptide fragments such as CSVTCG, CSVTCR, CSTSCR, CSTSCG, CRVTCG, RCRVTCG, ASVTAR, CSVTCK, CSTSCK, CSRTCG, CRTSCG, CRVTC, CSTSC or PCSVTCR or a compound such as AGM-1470 (TMP-470), Batimastat, Suramin, Galardin, Mitoflaxone, SU 1433, B-428, B-623 or SU 1498. The particulate embolic agent may also carry radionuclides for therapy and/or diagnosis such as those selected from the radioisotopes of Sc, Fe, Pb, Ga, Y, Bi, Mn, Cu, Cr, Zn, Ge, Mo, Tc, I, Ru, In, Sn, Sr, Sm, Lu, Sb, W, Re, Po, Ta and Tl. Preferred radionuclides include ^{99m}Tc, ¹²³I and ¹¹¹In. The radionuclides are either entrapped within the particles or complexed by an appropriate chelating moiety that is entrapped within or conjugated to the particles.

The pharmaceutical compositions may contain other conventional pharmaceutical excipients, e.g., viscosity modifiers, pH modifiers, chelating agents and salts thereof, salts of plasma cations (e.g., Na, Ca, K or Mg salts), etc.

The pharmaceutical compositions may be administered in a conventional fashion. The conventional use of embolus generating agents is described for example by Tomura et al. in Acta Radiologica 37: 52-56 (1996), Berenstein et al. in Radiology 145: 846 (1982) and Jack et al. in Nucl. Med. Biol 13: 235-243 (1986), the disclosures of which are incorporated herein by reference.

The invention will now be described further with reference to the accompanying drawings and the following non-limiting Examples:

### Example 1

### Treatment of Polyvinyl Alcohol Particles with Pluronic F68(Poloxamer 188)

0.1g of Ultra Ivalon polyvinyl alcohol particles between 150 and 250 microns in effective diameter suspended in 4 ml of saline were diluted with 11 ml of Omnipaque 350 (350 mg I/ml) and 150 mg of F68.

The resulting suspension was isodense and did not settle or float during the course of the experiment. This suspension was then tested in a controlled press (LR 10K, Chatillon Instruments) for back pressure during displacement relative to a PVA control suspension without the surfactant.

The test samples of the composition of Example 1 were loaded into a 3 ml syringe supplied with a 22 gauge 1½ inch long needle and were expelled at a rate of 0.3 ml/sec. Five samples were tested in this way as were five samples of a corresponding suspension from which the surfactant was omitted. The flow was measured as plunger force (in lbs force) against plunger displacement (in inches). The results for the surfactant-free samples are shown in Figure 1 while those for the surfactant-containing samples are shown in Figure 2.

### Example 2

### Treatment of Polyvinyl Alcohol Particles with Tween 80

0.1 g of Ultra Ivalon polyvinyl alcohol particles between 150 and 250 microns in effective diameter suspended in 4 ml of saline were diluted with 11 ml of Omnipaque 350 (350 mg I/ml) and enough Tween 80 that the resulting suspension was 1 mg/ml of Tween 80. The resulting suspension was isodense and did not settle or float during the course of the experiment. This suspension was then tested in a controlled press (LR 10K, Chatillon Instruments) for back pressure during displacement relative to a PVA control suspension without the surfactant. The Tween 80 treated particles were significantly enhanced with respect to ease of flow through the needle as opposed to the control PVA particles without surfactant.

### Example 3

### Treatment of Polyvinyl Alcohol Particles with Tween 80. Effect on Transit Through StarFast Interventional Catheters

0.1 g of Ultra Ivalon polyvinyl alcohol particles between 50 and 150 microns in effective diameter suspended in 4 ml of saline were diluted with 10 ml of Omnipaque 240 (240 mg I/ml) and 2 mg of Tween 80. The resulting suspension was isodense and did not settle or float during the course of the experiment. This suspension was then tested in a controlled press (LR 10K, Chatillon Instruments) for back pressure during displacement relative to a PVA control suspension without the surfactant. The Tween 80 treated particles were significantly enhanced with respect to ease of flow through the StarFast Catheter as opposed to the control PVA particles without surfactant. Force displacement curves (see Figures 3 and 4) show fundamental forces of approximately 6.5 Newtons over the first 15 mm of syringe plunger displacement for either the surfactant treated or the control PVA. However, further displacement (at 200 mm/min) of the syringe plunger in the case of the control PVA particles always resulted in force excursions of greater than 15 N and often greater than 25 N. Further displacement of the Tween 80 treated particles resulted in smaller, transient force excursions of 8 to 12 N with none greater than 15 N. Graphical representation of the data (Figures 3 and 4) clearly show the benefit of the surfactant in facilitating the flow of the PVA particles through the StarFast catheter.

## Claims

1. The use of a physiologically tolerable surfactant for the manufacture of an embolus generating pharmaceutical composition comprising a suspension of a particulate embolic agent in a physiologically tolerable liquid suspension medium, for use in a method of therapy or diagnosis.

2. A pharmaceutical composition comprising a suspension of a particulate embolic agent in a physiologically tolerable liquid suspension medium further containing a physiologically tolerable surfactant.

3. A sterile pharmaceutical concentrate comprising a particulate embolic agent and a surfactant.

4. A method of treatment of the human or vascularized animal to create an embolus therein, said method comprising administering to said body an effective amount of an embolus generating pharmaceutical composition as defined in claim 2.
